# EUROPEAN PATENT APPLICATION

(11) **EP 4 134 365 A1**
(43) Date of publication of application: **15.02.2023**
(21) Application number: 21191013.8
(22) Date of filing: 12.08.2021
(51) Int. Cl.: C07D 319/12, B01J 19/00

(54) **A PLANT AND AN EFFICIENT PROCESS FOR PRODUCING LACTIDE FROM LACTIC ACID**

(71) Applicant: Sulzer Management AG, 8401 Winterthur (CH)
(72) Inventor: DISTANTE, Francesco, 8404 Winterthur (CH); KEHRER, Florian, 8307 Effretikon (CH); VON SCALA REICHENBACH DE SOUSA, Claudia, 8606 Greifensee (CH)
(74) Representative: Henkel & Partner mbB

(57) **Abstract**

A process of producing lactide from lactic acid comprises the steps of:
a) feeding lactic acid, a hydrocarbon solvent and at least one catalyst into a reactor of a reactor system so as to form a reaction mixture in the reactor,
b) reacting the reaction mixture in the so that at least a part of the lactic acid is converted to lactide and water, while distilling at least a part of the reaction mixture so as to remove an azeotrope of water and the hydrocarbon solvent from the reactor,
c) separating at least a part of the water from the azeotrope removed from the reactor in step b),
d) separating at least a part of the lactide from the reaction mixture,
wherein the at least one catalyst comprises a homogeneous catalyst comprising an organometallic compound selected from the group consisting of tin alkoxides, zinc alkoxides, aluminum alkoxides, titanium alkoxides, tin carboxylates, zinc carboxylates, aluminum carboxylates, titanium carboxylates and arbitrary mixtures of two or more of these compounds.

## Description

The present invention relates to a process and to a plant of producing and preferably of continuously producing lactide from lactic acid.

Lactic acid is the monomer of polylactic acid. Homo- and copolymers of lactic acid are of particular interest, because they can be obtained from renewable resources and are mostly compostable and/or biodegradable. Moreover, the technological and physiochemical properties of these polymers come quite close to the properties of those polymers derived from fossil-based resources, which explains why these polymers are regarded as highly promising substitutes for the latter. Furthermore, homo- and copolymers of lactic acid have a wide range of applications. For instance, polylactic acid is used in the biomedical field in chirurgical implants, in films, such as e.g. in packagings, in fibers, such as e.g. for garments, in hygienic articles, in carpets and in disposable plastic products, such as e.g. disposable cutlery or containers. In addition, polylactic acid has found wide application in composite materials, such as in fiber-reinforced plastics. Polylactic acid copolymers are interesting alternatives to the polylactic acid homopolymers, since by appropriate selection of the comonomers and their relative amounts to each other and by adjusting an appropriate molecular weight, certain properties of the copolymers may be tailored to the intended use.

Generally, two alternative principal methods are known for synthesizing polylactic acid homo- or copolymers. The first principal method is the direct polycondensation of lactic acid optionally together with one or more comonomers, such as caprolactone and/or glycolide, to the respective homopolymer or copolymer, respectively, such as the direct polycondensation of lactic acid to polylactic acid homopolymer. However, this principal method only leads to low molecular weight (co)polymers and is thus limited to specific (co)polymers.

The second principal method known for synthesizing polylactic acid homo- or copolymers is the ring-opening-polymerization of lactide (which is the cyclic diester of lactic acid) optionally with one or more other cyclic esters, such as glycolide (which is the cyclic diester of glycolic acid), lactones or the like. This is the preferred method nowadays for the industrial production of polylactic acid homo- or copolymers. The lactide may be produced by condensation of two lactic acid molecules. Alternatively, the lactide may be produced by first oligomerizing lactic acid and then subjecting the oligomer to a depolymerization reaction. For instance, the lactide is prepared by fermentation of carbohydrates from biomass, such as starch, sugar or corn resulting in lactic acid, by then oligomerizing the lactic acid and by afterwards subjecting the oligomers to the depolymerization reaction. Likewise, the optional other cyclic diester comonomers may be produced by condensation of two hydroxy acid molecules into a cyclic diester, such as condensation of glycolic acid to glycolide, or by intramolecular esterification of an aliphatic hydroxy acid, such as in the case of a lactone, for instance caprolactone. After purification, the lactide and the optional one or more other cyclic diesters of hydroxy acid as comonomer(s) are then polymerized in the presence of a catalyst and optionally an initiator to form high molecular weight polymer. The unreacted (co)monomers have to be removed after the polymerization to a final concentration of less than at least 0.5 % by weight, in order to obtain a product of marketable quality.

The synthesis of lactide from lactic acid by condensation of two lactic acid molecules to lactide and water must be performed in the presence of a catalyst, since otherwise the conversion rates are unacceptably low. Moreover, the catalyst must be selective in a sense that it increases the conversion rate of two lactic acid molecules to lactide and water compared with the conversion rate of the thermodynamically favored condensation of lactic acid to lactic acid oligomers. For this purpose, usually zeolite catalysts are typically used, such as acidic zeolite, since they fulfil the aforementioned requirements.

For instance, Dusselier et al. describe in Science, 2015, volume 349, pages 78 to 80 a process for producing lactide from lactic acid using an acidic zeolite and preferably H-Beta zeolite catalyst. More specifically, the respective process comprises reacting a mixture comprising lactic acid, water, o-xylene as solvent and H-Beta zeolite catalyst at 110°C under continuous reflux effected by a water-solvent distillation, which removes a part of the water produced during the reaction in order to favor the reaction. The specific zeolite catalyst shall have a high selectivity so as to obtain a high conversion rate of lactic acid to lactide with comparably small amounts of by-products, such as linear lactic acid dimer and other linear lactic acid oligomers. Furthermore, the conversion rate of lactic acid to lactide shall be increased by the combination of using zeolite catalyst and a comparably high boiling solvent, namely o-xylene having a boiling point of about 139°C, which shall lead to a higher conversion rate of lactic acid to lactide than a lower boiling solvent, such as toluene. The reaction mixture is continuously withdrawn from the reactor and then subjected to a liquid-liquid extraction in order to separate the reaction mixture into an aqueous phase fraction comprising water and the byproducts and into an organic lactide rich fraction, which is then crystallized for separating the lactide from the solvent. The fraction removed from the reactor by the water-solvent distillation is subjected to a phase separation in a settler in order to separate this fraction into water and solvent, which is recycled into the reactor. However, this process has several disadvantages. Firstly, zeolite is not soluble in the solvent and is thus in the process a heterogenous catalyst. This has the drawback that the zeolite catalyst needs to be regenerated comprising separation from the reaction mixture and then washing of the catalyst involving additional solvents and separations. Moreover, since the zeolite is not soluble in the solvent, the zeolite catalyst does not only act on the solvent phase, but also on the aqueous phase including the lactic acid oligomers and other byproducts thus unnecessarily generating more byproducts than in principle necessary. Another drawback is that according to this document the zeolite catalyst requires the use of o-xylene as solvent in order to obtain a sufficiently high conversion rate of lactic acid to lactide. However, o-xylene has a comparatively low solubility for lactide and a significant solubility for byproducts, such as lactic acid dimer and other lactic acid oligomers, so that a certain amount of lactide is lost in the aqueous phase obtained in the liquid-liquid extraction and the solvent phase obtained in the liquid-liquid extraction contains significant amounts of the byproducts.

In view of this, the object underlying the present invention is to provide a process of producing lactide from lactic acid, which allows to fast obtain lactide with a high purity and with a high yield at minimal capital cots for the plant and with minimal operational costs.

In accordance with the present invention this object is satisfied by providing a process of producing lactide from lactic acid comprising the steps of:
a) feeding lactic acid, a hydrocarbon solvent and at least one catalyst into a reactor of a reactor system so as to form a reaction mixture in the reactor,
b) reacting the reaction mixture in the reactor so that at least a part of the lactic acid is converted to lactide and water, while distilling at least a part of the reaction mixture so as to remove an azeotrope of water and the hydrocarbon solvent from the reactor,
c) separating at least a part of the water from the azeotrope removed from the reactor in step b),
d) separating at least a part of the lactide from the reaction mixture,
wherein the at least one catalyst comprises a homogeneous catalyst comprising an organometallic compound selected from the group consisting of tin alkoxides, zinc alkoxides, aluminum alkoxides, titanium alkoxides, tin carboxylates, zinc carboxylates, aluminum carboxylates, titanium carboxylates and arbitrary mixtures of two or more of these compounds.

This solution bases on the surprising finding that by using a homogeneous catalyst comprising an organometallic compound selected from the group consisting of tin alkoxides, zinc alkoxides, aluminum alkoxides, titanium alkoxides, tin carboxylates, zinc carboxylates, aluminum carboxylates, titanium carboxylates and arbitrary mixtures of two or more of these compounds, in a process comprising the steps of feeding lactic acid, a hydrocarbon solvent and at least one catalyst into a reactor of a reactor system so as to form a reaction mixture in the reactor, b) reacting the reaction mixture in the reactor so as to react at least a part of the lactic acid to form lactide and water, while distilling at least a part of the reaction mixture so as to remove an azeotrope of water and the hydrocarbon solvent from the reactor, c) separating at least a part of the water from the azeotrope removed from the reactor in step b), and d) separating at least a part of the lactide from the reaction mixture, lactide is not only obtained in a fast process with a high purity, but also with a high yield in an efficient manner. In particular, this process is operable with low operational costs and only requires a plant with low capital costs. The use of a homogeneous catalyst comprising at least one organometallic compound selected from the group consisting of tin alkoxides, zinc alkoxides, aluminum alkoxides, titanium alkoxides, tin carboxylates, zinc carboxylates, aluminum carboxylates, titanium carboxylates and arbitrary mixtures of two or more of these compounds has the advantage that it has a particular high selectivity, thus leading to a high conversion rate of lactic acid to lactide with very low amount of byproducts, such as linear lactic acid dimer and other lactic acid oligomers. This, among others, is due to the fact that the catalyst is selectively soluble in hydrocarbon solvents, such as toluene, but not in water, so that it performs its catalytical action only towards the species soluble in the hydrocarbon solvent, leaving the water soluble species, namely lactic acid oligomers and acidic impurities, far from the reactive environment. In addition, in difference to heterogeneous catalysts, like zeolites, the homogeneous catalyst used in the process in accordance with the present invention does not need any regeneration process to be performed with additional solvents and separations, since it is easily separable from the hydrocarbon solvent by distillation and may be directly re-used. In addition, these catalysts, such as for instance tin octoate (which is also known as tin octonoate, tin(II) 2-ethylhexanoate or stannous octoate (Sn(Oct)₂)), are mostly FDA approved catalysts and thus toxicologically not critical. A further advantage of the process in accordance with the present invention is that it may be performed instead with high boiling hydrocarbon solvents as in the aforementioned prior art, at an elevated pressure of for instance 0.2 MPa as well as with low boiling hydrocarbon solvents, such as toluene having a particular high selectivity, i.e. an excellently high solubility for lactide and a particular low solubility for byproducts, such as lactic acid oligomers. Therefore, the lactide is nearly completely included in the organic solvent phase of the reaction mixture, whereas the byproducts are nearly completely retained in the aqueous phase of the reaction mixture. Finally, the process in accordance with the present invention is flexible and may be performed batchwise, semi-continuously or continuously.

Homogeneous catalyst in the sense of the present invention means any catalyst that is present in the same phase as the reactant molecules, i.e. in the reaction mixture including the hydrocarbon solvent. In contrast to this, heterogeneous catalysts are in a different phase than one or more of the reactants and often act as a surface on which the reaction can occur. Thus, a homogeneous catalyst in the sense of the present invention is any non-solid catalyst having a high solubility in the used hydrocarbon solvent so that it dissolves during the reaction in the hydrocarbon solvent and not in the aqueous phase. Therefore, in accordance with the present invention the catalyst used in step a) has a solubility in the used hydrocarbon phase at ambient temperature, i.e. at 23°C, as well as at ambient pressure of at least 50% by weight, preferably of at least 75% by weight and more preferably of at least 95% by weight. In particular, the homogeneous catalyst has a higher solubility in the hydrocarbon solvent than in water. Preferably, the solubility of the catalyst used in step a) at 23°C in water is less than 10% by weight, more preferably is less than 5% by weight and most preferably is less than 1% by weight. Usually, the homogeneous catalyst is not at all soluble in water, but for instance decomposes when it is in contact with water. For instance, tin octonoate, which is the most preferred homogeneous catalyst of the present invention, dissociates in water into 2-ethylhexanoate and Sn(ll) ions and the Sn(II) ions, which spontaneously oxidize in water to form the insoluble inorganic solid Sn(IV)O₂. The use of homogeneous catalyst, in contrast to solid, heterogeneous catalysts, like zeolites, avoids the steps of adsorbing the reactants before the reaction and desorbing the products after the reaction, which are usually mass transfer limited.

In accordance with the present invention, the catalyst fed into the reactor in step a), i.e. the catalyst included in the reactor during step b), comprises at least one organometallic compound selected from the group consisting of tin alkoxides, zinc alkoxides, aluminum alkoxides, titanium alkoxides, tin carboxylates, zinc carboxylates, aluminum carboxylates, titanium carboxylates and arbitrary mixtures of two or more of these compounds. Preferably, the catalyst comprises at least 50%by weight of the aforementioned catalyst(s), more preferably at least 80%by weight of the aforementioned catalyst(s), even more preferably at least 90%by weight of the aforementioned catalyst(s), still more preferably at least 95%by weight of the aforementioned catalyst(s) and most preferably consists of the aforementioned catalyst(s). Thus, most preferably no other catalyst than one or more of the aforementioned organometallic compounds is used in steps a) and b).

Good results are in particular obtained, when the at least one catalyst comprises or consists of at least one organometallic compound being selected from the group consisting of tin carboxylates, zinc carboxylates, aluminum carboxylates, titanium carboxylates and arbitrary mixtures of two or more of these compounds. More preferably, all of the aforementioned carboxylates are organometallic compounds containing one or more C2-i4-carboxylate groups and more preferably one or more C₆₋₁₄-carboxylate groups. Even more preferably, the at least one catalyst comprises or consists of at least one tin carboxylate, preferably a tin carboxylate comprising one or more C₂₋₁₄-carboxylate groups and more preferably one or more C6-14-carboxylate groups. Still more preferably, the at least one catalyst comprises or consists of tin octoate (i.e. tin 2-ethylhexanoate), tin oxalate or dibutyltin dilaurate. Most preferably, the at least one catalyst comprises or consists of tin octoate. Tin octoate has the advantage of being FDA approved. Moreover, it is often used as catalyst in ring-opening polymerization reactions of lactide to poly lactic acid, which allows to easily integrate the process of producing lactide from lactic acid in accordance with the present invention with a subsequent process of reacting the so obtained lactide to poly lactic acid biopolymer.

In a further development of the idea of the present invention it is proposed that the content of the catalyst in the reaction mixture, which is fed in step a) into the reactor, is, based on the dry content of lactic acid in the reaction mixture, 0.01 to 1.00% by mole, preferably 0.02 to 0.50% by mole, more preferably 0.05 to 0.30% by mole, still more preferably 0.05 to 0.20% by mole and most preferably 0.08 to 0.12% by mole.

In accordance with the present invention, in step a) lactic acid, a hydrocarbon solvent and at least one catalyst are fed into a reactor of the reactor system so as to form a reaction mixture in the reactor. All of the aforementioned components may be added as one mixture or separately from each other into the reactor. In addition, further components may be fed into the reactor, such as preferably water and optionally one or more recycle streams, such as an aqueous stream including minor amounts of lactic acid and byproducts obtained by the purification of the water obtained in step d). Further preferably, all components are mixed together, before being fed as one mixture in step a) into the reactor. For instance, the hydrocarbon solvent may be mixed with the catalyst, before an aqueous solution of lactic acid in water and one or more optional recycle streams are mixed so as to obtain the reaction mixture.

Preferably, in step a) a mixture comprising lactic acid, water, a hydrocarbon solvent and at least one catalyst is fed into the reactor. The content of lactic acid in the aqueous part of the reaction mixture preferably amounts, based on the sum of lactic acid and water, to 60 to 80% by weight and the content of lactic acid in the reaction mixture preferably amounts, based on the content of hydrocarbon solvent, to 15 to 30% by weight.

In principle, the present invention is not particularly limited concerning the chemical nature of the hydrocarbon solvent, as long as it fulfils together with the catalyst the aforementioned solubility for a homogeneous catalyst.

Moreover, it is preferred that the hydrocarbon solvent used in the process in accordance with the present invention has a high solubility for lactide. More specifically, the solubility of lactide in the hydrocarbon solvent is at ambient temperature, i.e. at 23°C, as well as at ambient pressure at least 2% by weight, preferably of at least 3% by weight, more preferably of at least 4% by weight, yet more preferably of at least 4.5% by weight, still more preferably of at least 6% by weight and more preferably at least 7.5% by weight.

Good results are in particular obtained with benzene or a benzene derivative, such as any alkyl-substituted benzene, such as toluene, o-, m- or p-xylene, trimethylbenzene, tetramethylbenzene, pentamethybenzene, hexamethylbenzene, monoethylbenzene, diethylbenzene, triethylbenzene, tetraethylbenzene, pentaethylbenzene, hexaethylbenzene, monopropylbenzene, dipropylbenzene, tripropylbenzene, propylbenzene, pentapropylbenzene, hexapropylbenzene and combinations thereof.

In a further development of the idea of the present invention it is suggested that the hydrocarbon solvent is an aromatic hydrocarbon solvent, i.e. one hydrocarbon compound or a mixture of two or more hydrocarbon compounds, each of which having a boiling point of 80 to 130°C, more preferably of 90 to 120°C and still more preferably of 100 to 120°C.

Most preferably, the hydrocarbon solvent is toluene. Toluene not only has a very high selectivity, i.e. an excellently high solubility for lactide and a particular low solubility for byproducts, such as lactic acid oligomers, but is also comparably cheap and in particular significantly cheaper than high-boiling point solvents, such as xylene and isobutylbenzene.

In accordance with a particular preferred embodiment of the present invention, the reaction mixture is reacted in step b) of the process at a temperature of 100 to 180°C, more preferably at a temperature of 110 to 160°C, still more preferably at a temperature of 120 to 160°C, even more preferably at a temperature of 130 to 150°C and most preferably at a temperature of 135 to 145°C. These temperatures are particularly preferred if toluene is used as hydrocarbon solvent.

Moreover, it is preferred that the reaction mixture is reacted in step b) of the process at a pressure of 0.10 to 0.40 MPa, more preferably at a pressure of 0.15 to 0.30 MPa, still more preferably at a pressure of 0.15 to 0.25 MPa, even more preferably at a pressure of 0.18 to 0.22 MPa and most preferably at a pressure of 0.19 to 0.21 MPa. These pressures are particularly preferred if toluene is used as hydrocarbon solvent.

Particular good results are obtained, when both aforementioned embodiments are combined, namely the reaction mixture is reacted in step b) of the process at a temperature of 100 to 180°C and at a pressure of 0.10 to 0.40 MPa, more preferably at a temperature of 110 to 160°C and at a pressure of 0.15 to 0.30 MPa, still more preferably at a temperature of 120 to 160°C and at a pressure of 0.15 to 0.25 MPa, even more preferably at a temperature of 130 to 150°C and at a pressure of 0.18 to 0.22 MPa and most preferably at a temperature of 135 to 145°C and at a pressure of 0.19 to 0.21 MPa. These temperature and pressure conditions are in particular preferred if toluene is used as hydrocarbon solvent. Thereby the aforementioned solubility advantages of toluene, i.e. very high solubility for lactide and very low solubility for byproducts, are coupled with high-temperature and highpressure conditions, which are advantageous, because the kinetics of the reaction of lactic acid to lactide and water are driven by temperature.

The present invention is not particularly limited to the number and kind of reactors) comprised in the rector system. For instance, the reactor may comprise one or more continuous stirred-tank reactors, one or more loop reactors, one or more plug flow reactors and arbitrary combinations of one or more of the above mentioned reactors. Preferably, the reactor, into which in step a) lactic acid, a hydrocarbon solvent and at least one catalyst is fed, of the reactor system is a continuous stirred-tank reactor or a plug flow reactor. Most preferably, the reactor into which in step a) lactic acid, a hydrocarbon solvent and at least one catalyst is fed, of the reactor system is a continuous stirred-tank reactor.

The distillation in step b) may be realized in a distillation column, which is preferably arranged on top of the reactor which is more preferably a continuous stirred-tank reactor.

In accordance with a preferred embodiment of the present invention, the reactor system comprises one reactor, i.e. no further reactor, and more preferably as one reactor a continuous stirred-tank reactor. In this embodiment, the reaction mixture is withdrawn from the reactor and is then subjected to the separation of step d).

In accordance with an alternative embodiment of the present invention, the reactor system comprises two reactors in series to each other. Preferably, the first reactor, into which in step a) lactic acid, a hydrocarbon solvent and at least one catalyst is fed, is a continuous stirred-tank reactor, wherein the second reactor downstream of the first reactor is a continuous stirred-tank reactor or a plug flow reactor and preferably a continuous stirred-tank reactor. In this embodiment, the reaction mixture is withdrawn from the first reactor and led into the second reactor, before reaction mixture is withdrawn from the second reactor and subjected to the separation of step d).

In a further development of the idea of the present invention, the reaction mixture is reacted in step b) in the reactor system for 0.1 to 10 hours, preferably for 1 to 5 hours, more preferably for 2 to 4 hours and most preferably for 2.5 to 3.5 hours. This reaction time is the sum of the reaction times within all reactors comprised in the reactor system. Thus, if the reactor system comprises three reactors, the residence time of the reaction mixture in the first reactor plus the residence time of the reaction mixture in the second reactor plus the residence time of the reaction mixture in the third reactor, is the total time for which the reaction mixture is reacted in step b) in the reactor system. Reacting, in this connection, means to incubate the reaction mixture for a certain residence time in the reactor.

Good results are in the aforementioned embodiment in particular obtained, when the reaction mixture is reacted in step b) of the process at a temperature of 110 to 160°C and at a pressure of 0.15 to 0.30 MPa for 0.1 to 10 hours, more preferably at a temperature of 120 to 160°C and at a pressure of 0.15 to 0.25 MPa for 1 to 5 hours, even more preferably at a temperature of 130 to 150°C and at a pressure of 0.18 to 0.22 MPa for 2 to 4 hours and most preferably at a temperature of 135 to 145°C and at a pressure of 0.19 to 0.21 MPa for 2.5 to 3.5 hours.

In step d) of the process of the present invention, at least a part of the lactide is separated from the reaction mixture, after the reaction mixture has been withdrawn from the reactor system, i.e. from the most downstream reactor of the reactor system. This may be achieved by separating the reaction mixture for instance driven by gravitation into an organic phase containing the hydrocarbon solvent and the lactide and into an aqueous phase containing water and the byproducts, before the lactide is separated from the organic phase by one or more separation techniques, such as crystallization, distillation, absorption or any arbitrary combinations thereof. The obtained aqueous phase may be also further purified with any of separation technique, such as one or more of the aforementioned separation techniques, so as to obtain pure water and a concentrated aqueous fraction containing the byproducts. The latter may be recycled into the feed to the reactor system.

Thus, it is preferred that the separation of at least a part of the lactide from the reaction mixture of step d), i.e. from the reaction mixture which has been withdrawn from the most downstream reactor of the reactor system, comprises the following sub-steps:
d₁) separating the reaction mixture in a first vessel into an organic phase and an aqueous phase,
d₂) separating the organic phase obtained in step d₁) in a second vessel into a lactide fraction and into a hydrocarbon solvent fraction and
d₃) separating the aqueous phase obtained in step d₁) in a third vessel into water and a residual fraction comprising unreacted lactic acid and lactic acid oligomers.

In order to obtain an efficient and fast separation of the reaction mixture into the organic phase and the aqueous phase, it is proposed in a further development of the idea of the present invention that the first vessel used in step d₁) comprises one or more coalescers. For instance, the first vessel may be a column, which comprises one or more coalescers. Any kind of coalescer may be used for this purpose, such as any kind of electrostatic coalescer and/or any kind of mechanical coalescer. For instance, a mechanical coalescer comprising one or more structured corrugated metal or plastic sheets, a mechanical coalescer comprising two materials with widely varying surface-free energies to enhance the droplet coalescence of either phase, or a mechanical coalescer comprising cartridges made of a selection of fiber materials with the liquid flowing from the centers of the cartridges radially outwards may be used. Alternatively to one or more coalescers, the first vessel may comprise one or more mixer settlers.

In accordance with a further particularly preferred embodiment of the present invention, during the separation of step d₁) the organic phase is also washed with water in order to remove as much of byproducts and impurities from the organic phase as possible. Preferably, for this purpose the reaction mixture is fed into the first vessel of step d₁) at a first position, wherein water is fed into the first vessel of step d₁) at a second position and is distributed over the cross-sectional plane of the first vessel by means of one or more liquid distributors, wherein the second position is vertically above the first position. Thereby upwardly streaming organic phase, i.e. light phase, is washed with the water so that water soluble byproducts and impurities, such as lactic acid oligomers, are washed out of the organic phase.

In accordance with a further embodiment of the present invention, the second vessel used in step d₂) is a distillation column and the organic phase is distilled therein so as to obtain a lactide fraction and a hydrocarbon solvent fraction.

The lactide fraction obtained in step d₂) may be further purified in at least one further distillation step, in order to increase its purity to a desired level.

Preferably, the hydrocarbon solvent fraction obtained in step d₂) is recycled and fed into the reactor in step a) in order to minimize the amount of hydrocarbon solvent needed for the process.

Good results are in step d₃) are in particular obtained, when the third vessel used in step d₃) is a distillation column and the aqueous phase is distilled therein so as to obtain the water and the residual fraction comprising unreacted lactic acid and lactic acid oligomers. Alternatively a reverse osmosis (RO) membrane unit can be used for removal of water and recovery of unreacted lactic acid and its oligomers.

In accordance with a particular preferred embodiment of the present invention, a hydrolysis unit is arranged downstream of the first or of the third vessel being fluidly connected with the outlet for the fraction comprising unreacted lactic acid and lactic acid oligomers so that in a further step d₄) in the hydrolysis unit lactic acid oligomers are hydrolysed to shorter oligomers and lactic acid or even exclusively to lactic acid. The hydrolysis of step d₄) may be achieved by adding water and lactic acid to the residual fraction in the hydrolysis unit. Also, the hydrolysis unit may comprise an ion exchange catalyst, such as a solid acid catalyst or a solid base catalyst, preferably a solid acid catalyst, in order to enhance the reaction rate.

Preferably, the residual fraction comprising unreacted lactic acid and lactic acid oligomers obtained in step d₃) or, even more preferably, the residual fraction comprising unreacted lactic acid obtained in step d₄) is recycled and fed into the reactor in step a).

In step c) of the process in accordance with the present invention, at least a part of the water is separated from the azeotrope removed from the reactor in step b). For instance, the separation of at least a part of the water from the azeotrope performed in step c) may comprise the following sub-steps:
c₁) cooling the azeotrope partially or completely so as to obtain a liquid stream, wherein a part of the liquid stream solvent is recycled into the reactor, and
c₂) separating the part of the liquid stream which is not recycled into the reactor, into a water rich fraction and into a hydrocarbon solvent rich fraction.

The separation of step c₂) may be performed by decanting, by using one or more coalescers, by adsorption, by distillation or any other suitable separation technique. Preferably, the separation of step c₂) is done by decantation.

Preferably, the hydrocarbon rich solvent rich fraction obtained in step c₂) is fed together with the organic phase obtained in step d₁) into the second vessel of step d₂).

Moreover, it is preferred to feed the water rich fraction obtained in step c₂) together with the aqueous phase obtained in step d₁) into the third vessel of step d₃).

As set out above, the process in accordance with the present invention is flexible and may be performed batchwise, semi-continuously or continuously. Preferably, the process is performed continuously.

Another aspect of the present invention is a plant for producing lactide from lactic acid, which comprises:
- a reactor system comprising at least one reactor, wherein a distillation column is arranged on top of the reactor so as to be able to distill vapor generated during the operation in the reactor to an azeotrope of water and hydrocarbon, wherein the distillation column comprises an outlet line for the azeotrope of water and hydrocarbon solvent, and wherein the reactor system comprises a feed line and an outlet line for the reaction mixture,
- a separator being connected with the outlet line from the distillation column for separating at least a part of the water from the azeotrope removed through the outlet line and
- a separator being connected with the outlet line for the reaction mixture.

In accordance with a preferred embodiment of the present invention, the reactor system of the plant comprises one reactor, which is a continuous stirred-tank reactor, on the top of which the distillation column is arranged.

In accordance with an alternative embodiment of the present invention, the reactor system of the plant comprises two reactors in series to each other, wherein the first reactor, into which the feed line leads, is a continuous stirred-tank reactor, on the top of which the distillation column is arranged, wherein the second reactor downstream of the first reactor is a continuous stirred-tank reactor or a plug flow reactor and preferably a continuous stirred-tank reactor, on the top of which the distillation column is arranged, and wherein both reactors are connected via a line so as to remove during the operation reaction mixture from the first reactor into the second reactor.

In a further development of the idea of the present invention, it is suggested that the separator being connected with the outlet line for the reaction mixture comprises one or more coalescers. For instance, the separator may be a column, which comprises one or more coalescers. Any kind of coalescer may be used for this purpose, such as any kind of electrostatic coalescer and/or any kind of mechanical coalescer. For instance, a mechanical coalescer comprising one or more structured corrugated metal or plastic sheets, a mechanical coalescer comprising two materials with widely varying surface-free energies to enhance the droplet coalescence of either phase, or a mechanical coalescer comprising cartridges made of a selection of fiber materials with the liquid flowing from the centers of the cartridges radially outwards may be used.

In order to allow the organic phase to be washed with water during the separation of step d₁), it is preferred that the outlet line for the reaction mixture leads into the separator at a first position and a water inlet line leads into the separator at a second position, wherein the second position is vertically above the first position, and wherein the water inlet line is connected with a distributor for distributing the water over the cross-sectional plane of the separator.

A particularly preferred plant for producing lactide from lactic acid, comprises:
- a reactor system comprising a continuous stirred-tank reactor, on the top of which the distillation column is arranged, and downstream of the continuous stirred-tank reactor a further continuous stirred-tank reactor, on the top of which the distillation column is arranged, wherein both reactors are connected via a line so as to remove during the operation reaction mixture from the continuous stirred-tank reactor into the continuous stirred-tank reactor, wherein the distillation columns each comprise an outlet line for the azeotrope of water and hydrocarbon solvent, and wherein the reactor system comprises a feed line and an outlet line for the reaction mixture,
- a first separator being connected with the outlet line for the reaction mixture, wherein the first separator is a column comprising one or more electrostatic coalescers and/or one or more mechanical coalescers, wherein the outlet line for the reaction mixture leads into the column at a first position and a water inlet line leads into the separator at a second position, wherein the second position is vertically above the first position, and wherein the water inlet line is connected with a distributor for distributing the water over the cross-sectional plane of the separator, wherein the column further comprises an outlet line for organic phase and an outlet line for aqueous phase,
- a second separator being connected with the outlet lines from the distillation columns for separating at least a part of the water from the azeotrope removed through the outlet line for the azeotrope so as to obtain a water rich fraction and a hydrocarbon solvent rich fraction, wherein the second separator comprises an outlet line for water rich fraction and an outlet line for hydrocarbon solvent rich fraction, and
- a further distillation column, which comprises an inlet line being connected with the outlet line for organic phase of the first separator and which comprises an inlet line being connected with the outlet line for a hydrocarbon solvent rich fraction of the second separator, wherein the distillation column comprises an outlet line for lactide and an outlet line for hydrocarbon solvent, the latter of which being preferably connected with the feed line of the reactor system.

The plant of the aforementioned embodiment preferably further comprises another distillation column, which comprises an inlet line being connected with the outlet line for aqueous phase of the first separator and being connected with the outlet line for water rich fraction of the second separator, wherein the distillation column further comprises an outlet line for water and an outlet line for an aqueous byproduct containing phase. The latter outlet line may be connected with the feed line of the reactor system.

In accordance with a particular preferred embodiment of the present invention, a hydrolysis unit is arranged downstream of the other distillation column, wherein the hydrolysis unit is connected with the outlet line for the aqueous byproduct containing phase. The hydrolysis unit may comprise an inlet line for water and an inlet line for lactic acid or an inlet line for a mixture of water and lactic acid. In addition, the hydrolysis unit may comprise an ion exchange catalyst, such as a solid acid catalyst. Moreover, the hydrolysis unit may comprise an outlet line for a lactic acid and lactic acid oligomers containing stream, wherein the outlet line may be connected with the feed line of the reactor system.

Preferably, the plant of the aforementioned embodiment further comprises a third distillation column, which comprises an inlet line being connected with the outlet line for lactide of the first distillation column and which comprises an outlet line for lactide, an outlet line for hydrocarbon solvent and an outlet line for purge.

Subsequently, the present invention is described by means of illustrative, but not limiting figures, in which:

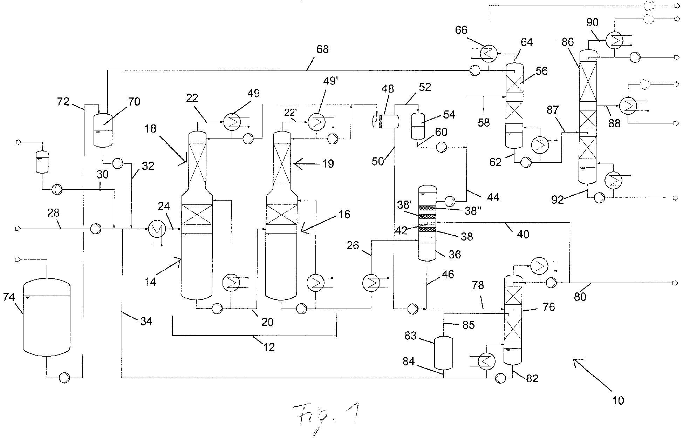

- Fig. 1: shows a schematic view of the plant in accordance with one embodiment of the present invention.

The plant 10 shown in fig. 1 comprises a reactor system 12, which comprises a first continuous stirred-tank reactor 14 and downstream of the first continuous stirred-tank reactor 14 a second continuous stirred-tank reactor 16. Both reactors 14, 16 are connected via a line 20 so that during operation of the plant 10 reaction mixture from the first continuous stirred-tank reactor 14 is led into the second continuous stirred-tank reactor 16. On the top of the first continuous stirred-tank reactor 14 a (first) distillation column 18 is arranged, which comprises an outlet line 22 for the azeotrope of water and hydrocarbon solvent. Likewise, on the top of the second continuous stirred-tank another (second) distillation column 19 is arranged, which comprises an outlet line 22' for the azeotrope of water and hydrocarbon solvent. The reactor system 12 comprises a feed line 24 and an outlet line 26 for the reaction mixture, wherein the feed line 24 is connected with an inlet line 28 for a lactic acid/water-mixture, with an inlet line 30 for catalyst, an inlet line 32 for hydrocarbon solvent and with a recycle line 34 for an aqueous fraction including byproducts of the reaction such as lactic acid oligomers and lactic acid.

Downstream of the reactor system 12, a first separator 36 is arranged, which is connected with the outlet line 26 for the reaction mixture from the second continuous stirred-tank reactor 16, wherein the outlet line 26 leads into the lower part of the first separator 36 at a first position. More specifically, the first separator 36 is a column comprising three mechanical coalescers 38, 38', 38". Furthermore, the first separator 36 comprises an inlet line 40 for wash water, which leads into the column of the first separator 36 at a second position, which is vertically above the first position. The column of the first separator 36 comprises a liquid distributor 42, which distributes during the operation of the plant 10 the water introduced into the column of the first separator 36 over the cross-sectional plane of the first separator 36. In addition, the column of the first separator 36 comprises an outlet line 44 for organic phase and an outlet line 46 for aqueous phase.

Moreover, the plant 10 comprises as second separator 48 a settler being connected with the outlet lines 22,22' from the distillation columns 18, 19 for separating at least a part of the water from the azeotrope removed through the outlet lines 22, 22' for the azeotrope via a condensers 49, 49' so as to obtain during the operation of the plant 10 a water rich fraction and a hydrocarbon solvent rich fraction. While the water rich fraction is withdrawn from the second separator 48 through outlet line 50, the hydrocarbon solvent rich fraction is withdrawn from the second separator 48 through outlet line 52, which is connected with a buffer tank 54.

Furthermore, the plant 10 comprises a third distillation column 56, which comprises an inlet line 58 being connected with the outlet line 44 for organic phase of the first separator 36. Also, the inlet line 58 is connected with the outlet line 60 of the buffer tank 54 for the hydrocarbon solvent rich fraction. The third distillation column 56 comprises an outlet line 62 for lactide and an outlet line 64 for hydrocarbon solvent. The latter outlet line 64 is connected via a condenser 66, via a recycle line 68, via a buffer tank 70 and via line 32 with the feed line 24 of the reactor system 12. Also, an inlet line 72 for fresh hydrocarbon solvent from buffer tank 74 leads into the buffer tank 70.

In addition, the plant 10 further comprises a fourth distillation column 76, which comprises an inlet line 78 being connected with the outlet line 46 for aqueous phase of the column of the first separator 36 and being connected with the outlet line 50 for water rich fraction of the second separator 48. The fourth distillation column 76 further comprises an outlet line 80 for water and an outlet line 82 for an aqueous byproduct containing phase. Downstream of the fourth distillation column 76, a hydrolysis unit 83 is arranged, which is connected via line 84 with the outlet line 82, and which further comprises an outlet line 85 leading back into the fourth distillation column 76. The outlet line 82 is further connected via the recycle line 34 for the aqueous fraction including byproducts of the reaction with the feed line 24 of the reactor system 12.

Finally, the plant 10 further comprises a fifth distillation column 86, which comprises an inlet line 87 being connected with the outlet line 62 for lactide of the third distillation column 56 and which comprises an outlet line for lactide 88, an outlet line 90 for hydrocarbon solvent and an outlet line 92 for purge.

During the operation of the plant 10, a reaction mixture comprising water, lactic acid, catalyst and hydrocarbon solvent is fed via feed line 24 into the first continuous stirred-tank reactor 14. The lactic acid contained in the reaction mixture reacts in the first continuous stirred-tank reactor 14 to lactide and water, wherein during the reaction continuously an azeotrope of water and hydrocarbon solvent is distilled in the first distillation column 18, which is withdrawn from the first distillation column 18 via the outlet line 22. The reaction mixture of the the first continuous stirred-tank reactor 14 is continuously withdrawn from the first continuous stirred-tank reactor 14 and led into the second continuous stirred-tank reactor 16, in which the reaction is continued. During the reaction continuously an azeotrope of water and hydrocarbon solvent is distilled in the second distillation column 19, which is withdrawn from the second distillation column 19 via the outlet line 22'. The reaction mixture of the second continuous stirred-tank reactor 16 is continuously withdrawn from the second continuous stirred-tank reactor 16 and led via line 26 into the column of the first separator 36. The reaction mixture is separated in the first separator 36 into an aqueous phase comprising water and byproducts/impurities from the reaction, and into an organic phase, which is washed by the water being fed into the column of the first separator 36 via line 40 and being distributed over the cross-sectional plane of the first separator 36 by the liquid distributor 42. Furthermore, the azeotrope of water and hydrocarbon solvent being withdrawn from the first and second distillation columns 18, 19 via lines 22, 22' is condensed in the condensers 49, 49'. A portion of the stream obtained thereby is recycled into the distillation columns 18, 19, whereas the remaining portion of the stream is led into the second separator 48 or settler, respectively. In the second separator 48, the stream separates into a water rich fraction and a hydrocarbon solvent rich fraction. While the water rich fraction is withdrawn from the second separator 48 through outlet line 50, the hydrocarbon solvent rich fraction is withdrawn from the second separator 48 through outlet line 52 and via the buffer tank 54 together with the organic phase obtained in the first separator 36 via line 58 into the third distillation column 56. The hydrocarbon solvent rich fraction is separated in the third distillation column 56 into a hydrocarbon solvent fraction as well as into a lactide fraction. While the lactide fraction is withdrawn from the third distillation column 56 via line 62 and further purified in the downstream distillation column 86, the hydrocarbon solvent rich fraction is withdrawn from the third distillation column 56 via line 64, condensed in the condenser 66 and returned via line 68, buffer tank 70, line 32 and feed line 24 into the first continuous stirred-tank reactor 14. The water rich fraction being withdrawn from the second separator 48 through outlet line 50 is led together with the aqueous phase being withdrawn from the column of the first separator 36 via line 78 into the distillation column 76. Water is removed from the distillation column 76 via line 80, whereas the aqueous byproduct containing phase is led from the distillation column 76 partially via line 84 into the hydrolysis unit 83 and from there via line 85 back into the distillation column 76, whereas the remaining part of the aqueous byproduct containing phase is led via lines 82, 34, 24 into the first continuous stirred-tank reactor 14. Lactic acid oligomers are reacted in the hydrolysis unit 83 to shorter lactic acid oligomers and/or to lactic acid, thus reducing the size and amount of lactic acid oligomers recycled via lines 34, 24 into the first continuous stirred-tank reactor 14.

### Reference numerals

- 10: Plant
- 12: Reactor system
- 14: First reactor/continuous stirred-tank reactor
- 16: Second reactor/ continuous stirred-tank reactor
- 18: (First) distillation column
- 19: (Second) distillation column
- 20: Line for reaction mixture
- 22, 22': Outlet line for azeotrope
- 24: Feed line into the continuous stirred-tank reactor
- 26: Outlet line for reaction mixture
- 28: Inlet line for a lactic acid/water-mixture
- 30: Inlet line for catalyst
- 32: Inlet line for hydrocarbon solvent
- 34: Recycle line
- 36: First vessel/first separator
- 38, 38', 38": Mechanical coalescers
- 40: Inlet line for wash water
- 42: Liquid distributor
- 44: Outlet line for organic phase
- 46: Outlet line for aqueous phase
- 48: Second separator
- 49, 49': Condenser
- 50: Outlet line for water rich fraction
- 52: Outlet line for hydrocarbon solvent rich fraction
- 54: Buffer tank
- 56: Second vessel/(third) distillation column
- 58: Inlet line of third distillation column
- 60: Outlet line of the buffer tank
- 62: Outlet line for lactide
- 64: Outlet line for hydrocarbon solvent
- 66: Condenser
- 68: Recycle line
- 70: Buffer tank
- 72: Inlet line for fresh hydrocarbon solvent
- 74: Buffer tank
- 76: Third vessel/(fourth) distillation column
- 78: Inlet line of fourth distillation column
- 80: Outlet line for water
- 82: Outlet line for an aqueous byproduct containing phase
- 83: Hydrolysis unit
- 84: Line to hydrolysis unit
- 85: Line from hydrolysis unit
- 86: (Fifth) distillation column
- 87: Inlet line to fifth distillation column
- 88: Outlet line for lactide
- 90: Outlet line for hydrocarbon solvent
- 92: Outlet line for purge

## Claims

1. A process of producing lactide from lactic acid comprising the steps of:
a) feeding lactic acid, a hydrocarbon solvent and at least one catalyst into a reactor (14) of a reactor system (12) so as to form a reaction mixture in the reactor (14),
b) reacting the reaction mixture in the reactor (14) so that at least a part of the lactic acid is converted to lactide and water, while distilling at least a part of the reaction mixture so as to remove an azeotrope of water and the hydrocarbon solvent from the reactor (14),
c) separating at least a part of the water from the azeotrope removed from the reactor (14) in step b),
d) separating at least a part of the lactide from the reaction mixture,
wherein the at least one catalyst comprises a homogeneous catalyst comprising an organometallic compound selected from the group consisting of tin alkoxides, zinc alkoxides, aluminum alkoxides, titanium alkoxides, tin carboxylates, zinc carboxylates, aluminum carboxylates, titanium carboxylates and arbitrary mixtures of two or more of these compounds.

2. The process in accordance with claim 1, wherein the at least one catalyst comprises or is a tin carboxylate, preferably a tin carboxylate comprising one or more C₂₋₁₄-carboxylate groups and more preferably one or more C₆₋₁₄-carboxylate groups, wherein the at least one catalyst is most preferably tin octoate.

3. The process in accordance with claim 1 or 2, wherein the hydrocarbon solvent is an aromatic hydrocarbon solvent having a boiling point of 80 to 130°C, preferably of 90 to 120°C and more preferably of 100 to 120°C, wherein preferably the hydrocarbon solvent is toluene.

4. The process in accordance with any of the preceding claims, wherein the reaction mixture is reacted in step b) at a temperature of 100 to 180°C, preferably at a temperature of 110 to 160°C, more preferably at a temperature of 120 to 160°C, even more preferably at a temperature of 130 to 150°C and most preferably at a temperature of 135 to 145°C.

5. The process in accordance with any of the preceding claims, wherein the reaction mixture is reacted in step b) at a pressure of 0.10 to 0.40 MPa, preferably at a pressure of 0.15 to 0.30 MPa, more preferably at a pressure of 0.15 to 0.25 MPa, even more preferably at a pressure of 0.18 to 0.22 MPa and most preferably at a pressure of 0.19 to 0.21 MPa.

6. The process in accordance with any of the preceding claims, wherein the reactor (14), into which in step a) lactic acid, a hydrocarbon solvent and at least one catalyst is fed, is a continuous stirred-tank reactor, wherein the azeotropic distillation in step b) is performed in a distillation column (18), which is arranged on top of the continuous stirred-tank reactor (14).

7. The process in accordance with any of the preceding claims, wherein the reactor system (12) comprises two reactors (14, 16) in series to each other, wherein the first reactor (14), into which in step a) lactic acid, a hydrocarbon solvent and at least one catalyst is fed, is a continuous stirred-tank reactor, wherein the second reactor (16) downstream of the first reactor (14) is a continuous stirred-tank reactor, and wherein the reaction mixture is withdrawn from the first reactor (14) and led into the second reactor (16), before reaction mixture is withdrawn from the second reactor (16) and subjected to the separation of step d).

8. The process in accordance with any of the preceding claims, wherein the reaction mixture is reacted in step b) in the reactor system (12) for 0.1 to 10 hours, preferably for 1 to 5 hours, more preferably for 2 to 4 hours and most preferably for 2.5 to 3.5 hours.

9. The process in accordance with any of the preceding claims, wherein the separation of at least a part of the lactide from the reaction mixture of step d) comprises the following sub-steps:
d₁) separating the reaction mixture in a first vessel (36) into an organic phase and an aqueous phase,
d₂) separating the organic phase obtained in step d₁) in a second vessel (56) into a lactide fraction and into a hydrocarbon solvent fraction and
d₃) separating the aqueous phase obtained in step d₁) in a third vessel (76) into water and a residual fraction comprising unreacted lactic acid and lactic acid oligomers.

10. The process in accordance with claim 9, wherein the first vessel (36) used in step d₁) is a column comprising one or more coalescers (38, 38', 38"), wherein preferably the first vessel (36) used in step d₁) is a column comprising one or more electrostatic coalescers (38, 38', 38") and/or one or more mechanical coalescers (38, 38', 38"), preferably one or more mechanical coalescers (38, 38', 38") comprising one or more structured corrugated metal or plastic sheets, one or more mechanical coalescers comprising two materials with widely varying surface-free energies to enhance the droplet coalescence of either phase and/ or one or more mechanical coalescers comprising cartridges made of a selection of fiber materials with the liquid flowing from the centers of the cartridges radially outwards.

11. The process in accordance with claim 9 or 10, wherein the second vessel (56) used in step d₂) is a distillation column (56) and the organic phase is distilled therein so as to obtain a lactide fraction and a hydrocarbon solvent fraction, wherein preferably the hydrocarbon solvent fraction obtained in step d₂) is recycled and fed into the reactor (14) in step a).

12. The process in accordance with any of the preceding claims, wherein the separation of at least a part of the water from the azeotrope performed in step c) comprises the following sub-steps:
c₁) cooling the azeotrope so as to obtain a liquid stream, and
c₂) separating the liquid stream into a water rich fraction and into a hydrocarbon solvent rich fraction.

13. A plant (10) for producing lactide from lactic acid comprising:
- a reactor system (12) comprising at least one reactor (14), wherein a distillation column (18) is arranged on top of the reactor (14) so as to be able to distill vapor generated during the operation in the reactor (14) to an azeotrope of water and hydrocarbon, wherein the distillation column (18) comprises an outlet line (22) for the azeotrope of water and hydrocarbon solvent, and wherein the reactor system (12) comprises a feed line (24) and an outlet line for the reaction mixture (26),
- a separator (48) being connected with the outlet line (22) from the distillation column for separating at least a part of the water from the azeotrope removed through the outlet line and
- a separator (36) being connected with the outlet line (26) for the reaction mixture.

14. The plant (10) of claim 13, wherein the reactor system (12) comprises one reactor (14), which is a continuous stirred-tank reactor (14), on the top of which the distillation column (18) is arranged, or, wherein the reactor system (12) comprises two reactors (14, 16) in series to each other, wherein the first reactor (14), into which the feed line leads, is a continuous stirred-tank reactor (14), on the top of which the distillation column (18) is arranged, wherein the second reactor (16) downstream of the first reactor (14) is a continuous stirred-tank reactor, on the top of which a distillation column (19) is arranged, and wherein both reactors (14, 16) are connected via a line (20) so as to remove during the operation reaction mixture from the first reactor (14) into the second reactor (16).

15. The plant (10) of claim 13 or 14, wherein the separator (36) being connected with the outlet line (26) for the reaction mixture comprises one or more coalescers (38, 38', 38"), wherein preferably the separator (36) comprises one or more coalescers (38, 38', 38") and preferably one or more electrostatic coalescers (38, 38', 38") and/or one or more mechanical coalescers (38, 38', 38"), and more preferably one or more mechanical coalescers (38, 38', 38") comprising one or more structured corrugated metal or plastic sheets, one or more mechanical coalescers (38, 38', 38") comprising two materials with widely varying surface-free energies to enhance the droplet coalescence of either phase and/ or one or more mechanical coalescers (38, 38', 38") comprising cartridges made of a selection of fiber materials with the liquid flowing from the centers of the cartridges radially outwards.
